# EUROPEAN PATENT SPECIFICATION

(11) **EP 4 093 313 B1**
(45) Date of publication and mention of the grant of the patent: **19.03.2025**
(21) Application number: 20838713.4
(22) Date of filing: 09.12.2020
(51) Int. Cl.: A61B 34/20, A61B 90/00, A61B 17/00, A61B 1/313

(54) **CATHETER TIP WITH INTEGRATED ELECTRONICS PACKAGE AND CATHETER INCORPORATING THE SAME**
KATHETERSPITZE MIT INTEGRIERTEM ELEKTRONIKPAKET UND KATHETER DAMIT
POINTE DE CATHÉTER À BOÎTIER ÉLECTRONIQUE INTÉGRÉ ET CATHÉTER L'INCORPORANT

(30) Priority: 10.03.2020 US 202062987574 P
(43) Date of publication of application: 30.11.2022
(73) Proprietor: St. Jude Medical, Cardiology Division, Inc., St. Paul MN 55117 (US)
(72) Inventor: LUPOTTI, Fermin Armando, Lake Forest, California 92630 (US); MEYERSON, Scott C., Ham Lake, Minnesota 55304 (US)
(74) Representative: Mathys & Squire
(86) International application number: PCT/US2020/064036
(87) International publication number: WO 2021/183196

(56) References cited:
- WO-A1-2018/220215
- WO-A1-2019/232256
- US-A1- 2007 293 721
- US-A1- 2008 009 745
- US-A1- 2019 380 589

## Description

### CROSS-REFERENCE TO RELATED APPLICATIONS

This application claims the benefit of United States provisional application no. 62/987,574, filed 10 March 2020.

### BACKGROUND

The present disclosure relates generally to catheters that are used in the human body. In particular, the present disclosure relates to an intravascular catheter that includes an integrated electronics package in its distal region, such as within its tip.

Catheters are used for an ever-growing number of procedures. For example, catheters are used for diagnostic, therapeutic, and ablative procedures, to name just a few examples. Typically, the catheter is manipulated through the patient's vasculature and to the intended site, for example, a site within the patient's heart.

For example, an ultrasound catheter is known from US 2008/0009745 A1. A catheter with merged optical tissue evaluation is known from US 2019/0380589 A1. A catheter handle with compliant circuit is known from WO 2019/232256 A1. US2007/293721 describes a small gauge steerable catheter including a locatable guide with a sheath, used as an enhancement to a bronchoscope.

### BRIEF SUMMARY

Disclosed herein is an intravascular catheter with the features according to claim 1 and a method of manufacturing a tip assembly for an intravascular catheter with the features according to claim 8. Preferred embodiments are given in the dependent claims.

The integrated electronics package can be a system on a chip, such as an application specific integrated circuit.

The plurality of magnetic localization elements can include a plurality of magnetic coils, which can be operably connected to the integrated electronics package. According to the invention, the plurality of magnetic localization elements include a plurality of solid-state magnetic localization elements, such as a plurality of anisotropic magnetoresistive sensors, and are mounted within the integrated electronics package.

The intravascular catheter further includes a shell.

It is contemplated that the plurality of magnetic localization elements can be incorporated into the integrated electronics package. For instance, a plurality of solid state magnetic localization elements can be incorporated into the integrated electronics package.

The integrated electronics package can include an application specific integrated circuit.

The foregoing and other aspects, features, details, utilities, and advantages of the present invention will be apparent from reading the following description and claims, and from reviewing the accompanying drawings.

### BRIEF DESCRIPTION OF THE DRAWINGS

Figure 1 is a perspective view of a representative catheter according to aspects of the instant disclosure.
Figure 2 illustrates a first embodiment of a tip assembly for an intravascular catheter as disclosed herein.
Figure 3 illustrates a second embodiment of a tip assembly according to the invention for an intravascular catheter as disclosed herein.

### DETAILED DESCRIPTION

Aspects of the instant disclosure relate to catheters with electronics packages integrated into their distal ends (e.g., positioned within their distal tip assemblies). Those of ordinary skill in the art will appreciate that the teachings herein can be applied to good advantage in connection with various types of catheters, including, but not limited to, intracardiac echocardiography (ICE) catheters. For instance, the teachings herein can be applied in connection with intravascular catheters as disclosed in United States application no. 15/948,818 ("the '818 application"). As another example, the teachings herein can be applied in connection with catheters such as those disclosed in United States patent application publication no. 2014/0275957 ("the '957 publication").

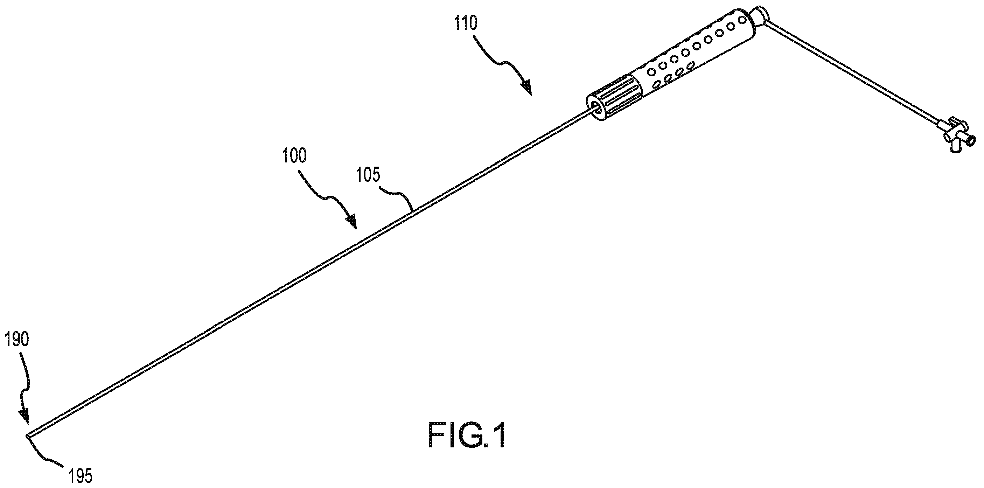

For purposes of illustration, Figure 1 depicts a perspective view of a representative catheter 100, including a shaft 105 having a proximal portion 110 and a distal portion 190, which terminates in a tip 195. Insofar as the basic construction of catheter 100 will be familiar to those of ordinary skill in the art, the details thereof will be omitted herein, except to the extent relevant to an understanding of the instant disclosure.

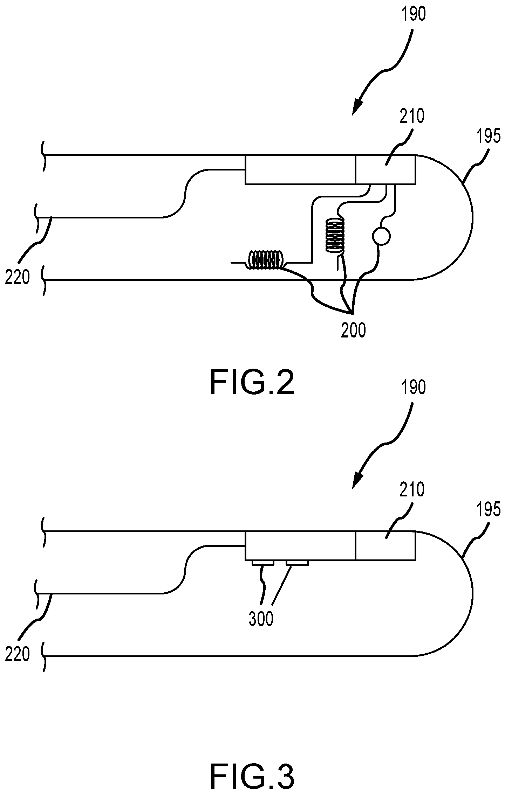

Figure 2 is a close-up view of distal portion 190, including tip 195, according to a first embodiment. A plurality of magnetic localization elements (e.g., magnetic coils 200) are disposed within distal portion 190 (which, for purposes of explanation, can also be referred to as a "shell"). Figure 2 depicts three orthogonal coils 200. As those of ordinary skill in the art will appreciate, these three orthogonal coils 200 allow tip 195 to be
localized with six degrees of freedom within a magnetic localization field generated by an electroanatomical mapping system such as the EnSite Precision^{™} cardiac mapping system of Abbott Laboratories (Chicago, IL).

Also shown within distal portion 190 is an integrated electronics package 210. In embodiments of the disclosure, integrated electronics package 210 is implemented as a system-on-a-chip (SoC), such as an application specific integrated circuit (ASIC) or a field programmable gate array (FPGA).

Integrated electronics package 210 includes a power supply, a pre-amplifier, a multiplexor, and electronics to drive imaging elements, such as transmitters and/or receivers. The imaging elements themselves are an ultrasonic transducer array and are included within integrated electronics package 210; alternatively, integrated electronics package 210 can include connections to imaging elements that are external to integrated electronics package 210 (embodiment not covered by the claimed invention).

Coils 200 are operably connected to integrated electronics package 210, for example to pass power from integrated electronics package 210 to coils 200 and to pass electromagnetic signals (*e.g.*, magnetic field measurements, control signals, and the like) between coils 200 and integrated electronics package 210.

Advantageously, because of the proximity between coils 200 and integrated electronics package 210, coils 200 can be made smaller than extant magnetic localization elements. For instance, extant coils 200 may be from about 0.5 cm to about 1 cm in length with diameters ranging from about 0.3 cm to about 0.5 cm. The proximity between coils 200 and integrated electronics package 210 according to the instant disclosure, however, allows these dimensions to be reduced by about 50%.

Additional cable connectors 220 are provided to connect integrated electronics package 210 (and thus coils 200) to a control unit. Although only one connector 220 is shown in the Figures, those of ordinary skill in the art will appreciate that multiple such connectors 220 can be used and/or bundled through shaft 105.

In another embodiment according to the invention and depicted in Figure 3, coils 200 are replaced by solid-state magnetic localization elements 300, which are mounted within integrated electronics package 210 (e.g., incorporated into the SoC or ASIC). For instance, solid-state magnetic localization elements 300 may be anisotropic magnetoresistive (AMR) sensors, such as the AFF811 sensor from Sensitec GmbH (Lahnau, Germany).

In either embodiment (that is, whether the magnetic localization elements are implemented as coils 200 or as solid state elements 300), the proximity between the magnetic localization elements and integrated electronics package 210 advantageously improves the quality of signal communication therebetween (*e.g.*, it offers an improved signal-to-noise ratio relative to extant catheters).

In addition, the burden and expense associated with assembly of catheter 100 is minimized through the incorporation of elements into integrated electronics package 210 (*e.g.,* requiring only a single bundle of connectors 220 (*e.g.,* wires) extending along catheter shaft 105; eliminating additional wiring between the magnetic localization sensors and the integrated electronics package when solid state elements 300 are used).

Although several embodiments have been described above with a certain degree of particularity, those skilled in the art could make numerous alterations to the disclosed embodiments without departing from the scope of this invention.

For example, integrated electronics package 210 can also include elements that sense the orientation of shaft 105, such as one or more solid state accelerometers.

All directional references (*e.g.*, upper, lower, upward, downward, left, right, leftward, rightward, top, bottom, above, below, vertical, horizontal, clockwise, and counterclockwise) are only used for identification purposes to aid the reader's understanding of the present invention, and do not create limitations, particularly as to the position, orientation, or use of the invention. Joinder references (*e.g.*, attached, coupled, connected, and the like) are to be construed broadly and may include intermediate members between a connection of elements and relative movement between elements. As such, joinder references do not necessarily infer that two elements are directly connected and in fixed relation to each other.

It is intended that all matter contained in the above description or shown in the accompanying drawings shall be interpreted as illustrative only and not limiting. Changes in detail or structure may be made without departing from the invention as defined in the appended claims.

## Claims

1. An intravascular catheter (100), comprising:
a catheter shaft (105) having a distal portion (190);
a plurality of magnetic localization elements disposed within the distal portion (190) of the catheter shaft (105); and
an integrated electronics package (210) disposed within the distal portion (190) of the catheter shaft (105), wherein the integrated electronics package (210) comprises:
a power supply;
a pre-amplifier;
a multiplexor;
an imaging element driver; and
imaging elements,
wherein the imaging elements are an ultrasonic transducer array which is included within the integrated electronics package (210) and
**characterised in that** the plurality of magnetic localization elements comprises a plurality of solid-state magnetic localization sensors (300) mounted within the integrated electronics package (210).

2. The intravascular catheter (100) according to claim 1, wherein the electronics package (210) comprises a system on a chip.

3. The intravascular catheter (100) according to claim 2, wherein the system on a chip comprises an application specific integrated circuit.

4. The intravascular catheter (100) according to claim 1, wherein the plurality of solid-state magnetic localization sensors comprises a plurality of anisotropic magnetoresistive sensors.

5. The intravascular catheter (100) according to any one of claims 1 to 4, wherein the distal portion of the catheter shaft (105) is a shell.

6. A method of manufacturing a tip assembly for an intravascular catheter (100), comprising:
forming a shell (190);
positioning an integrated electronics package (210) within the shell, wherein the integrated electronics package comprises:
a power supply;
a pre-amplifier;
a multiplexor;
an imaging element driver; and
imaging elements,
positioning a plurality of magnetic localization elements within the shell; and
operably connecting the plurality of magnetic localization elements to the integrated electronics package (210),
wherein the imaging elements are an ultrasonic transducer array which is included within the integrated electronics package (210),
**characterised in that** operably connecting the plurality of magnetic localization elements to the integrated electronics package (210) comprises incorporating the plurality of magnetic localization elements into the integrated electronics package (210),
wherein incorporating the plurality of magnetic localization elements into the integrated electronics package (210) comprises incorporating a plurality of solid-state magnetic localization sensors (300) into the integrated electronics package (210).

7. The method according to claim 6, wherein the integrated electronics package (210) comprises an application specific integrated circuit.

## Patentansprüche

1. Intravaskulärer Katheter (100), umfassend:
einen Katheterschaft (105) mit einem distalen Abschnitt (190);
eine Vielzahl von Magnetlokalisierungselementen, die innerhalb des distalen Abschnitts (190) des Katheterschafts (105) angeordnet sind; und
ein integriertes Elektronikpaket (210), das innerhalb des distalen Abschnitts (190) des Katheterschafts (105) angeordnet ist, wobei das integrierte Elektronikpaket (210) umfasst:
eine Energieversorgung;
einen Vorverstärker;
einen Multiplexer;
einen Bildgebungselementtreiber und
Bildgebungselemente,
wobei es sich bei den Bildgebungselementen um ein Ultraschallwandler-Array handelt, das innerhalb des integrierten Elektronikpakets (210) enthalten ist; und
**dadurch gekennzeichnet, dass** die Vielzahl von Magnetlokalisierungselementen eine Vielzahl von Festkörpermagnetlokalisierungssensoren (300) umfasst, die innerhalb des Elektronikpakets (210) montiert ist.

2. Intravaskulärer Katheter (100) nach Anspruch 1, wobei das Elektronikpaket (210) ein System-on-a-Chip umfasst.

3. Intravaskulärer Katheter (100) nach Anspruch 2, wobei das System-on-a-Chip eine anwendungsspezifische integrierte Schaltung umfasst.

4. Intravaskulärer Katheter (100) nach Anspruch 1, wobei die Vielzahl von Festkörpermagnetlokalisierungssensoren eine Vielzahl von anisotropen magnetoresistiven Sensoren umfasst.

5. Intravaskulärer Katheter (100) nach einem der Ansprüche 1 bis 4, wobei der distale Abschnitt des Katheterschafts (105) eine Schale ist.

6. Verfahren zur Herstellung einer Spitzenanordnung für einen intravaskulären Katheter (100), umfassend:
Bilden einer Schale (190);
Positionieren eines integrierten Elektronikpakets (210) innerhalb der Schale, wobei das integrierte Elektronikpaket umfasst:
eine Energieversorgung;
einen Vorverstärker;
einen Multiplexer;
einen Bildgebungselementtreiber und
Bildgebungselemente,
Positionieren einer Vielzahl von Magnetlokalisierungselementen innerhalb der Schale und
betriebsfähiges Verbinden der Vielzahl von Magnetlokalisierungselementen mit dem integrierten Elektronikpaket (210),
wobei es sich bei den Bildgebungselementen um ein Ultraschallwandler-Array handelt, das innerhalb des integrierten Elektronikpakets (210) enthalten ist;
**dadurch gekennzeichnet, dass** das betriebsfähige Verbinden der Vielzahl von Magnetlokalisierungselementen mit dem integrierten Elektronikpaket (210) ein Einbinden der Vielzahl von Magnetlokalisierungselementen in das integrierte Elektronikpaket (210) umfasst,
wobei das Einbinden der Vielzahl von Magnetlokalisierungselementen in das integrierte Elektronikpaket (210) ein Einbinden einer Vielzahl von Festkörpermagnetlokalisierungssensoren (300) in das integrierte Elektronikpaket (210) umfasst.

7. Verfahren nach Anspruch 6, wobei das integrierte Elektronikpaket (210) eine anwendungsspezifische integrierte Schaltung umfasst.

## Revendications

1. Cathéter intravasculaire (100), comprenant :
un tube de cathéter (105) comportant une partie distale (190) ;
une pluralité d'éléments magnétiques de localisation disposés à l'intérieur de la partie distale (190) du tube de cathéter (105) ; et
un boîtier électronique intégré (210) disposé à l'intérieur de la partie distale (190) du tube de cathéter (105), le boîtier électronique intégré (210) comprenant :
une alimentation électrique ;
un préamplificateur ;
un multiplexeur ;
un pilote d'éléments d'imagerie ; et
des éléments d'imagerie,
dans lequel les éléments d'imagerie sont un réseau de transducteurs ultrasoniques qui est inclus dans le boîtier électronique intégré (210) et
**caractérisé en ce que** la pluralité d'éléments de localisation magnétiques comprend une pluralité de capteurs de localisation magnétiques à semi-conducteurs (300) montés à l'intérieur du boîtier électronique intégré (210).

2. Cathéter intravasculaire (100) selon la revendication 1, dans lequel le boîtier électronique (210) comprend un système sur puce.

3. Cathéter intravasculaire (100) selon la revendication 2, dans lequel le système sur puce comprend un circuit intégré spécifique à l'application.

4. Cathéter intravasculaire (100) selon la revendication 1, dans lequel la pluralité de capteurs de localisation magnétiques à semi-conducteurs comprend une pluralité de capteurs magnétorésistifs anisotropes.

5. Cathéter intravasculaire (100) selon l'une quelconque des revendications 1 à 4, dans lequel la partie distale du tube de cathéter (105) est une coque.

6. Procédé de fabrication d'un ensemble pointe pour un cathéter intravasculaire (100), comprenant :
la formation d'une coque (190) ;
le positionnement d'un boîtier électronique intégré (210) à l'intérieur de la coque, le boîtier électronique intégré comprenant :
une alimentation électrique ;
un préamplificateur ;
un multiplexeur ;
un pilote d'éléments d'imagerie ; et
des éléments d'imagerie,
le positionnement d'une pluralité d'éléments de localisation magnétiques à l'intérieur de la coque ; et
le raccordement fonctionnel de la pluralité d'éléments de localisation magnétiques au boîtier électronique intégré (210),
dans lequel les éléments d'imagerie sont un réseau de transducteurs ultrasoniques qui est inclus dans le boîtier électronique intégré (210),
**caractérisé en ce que** le raccordement fonctionnel de la pluralité d'éléments de localisation magnétiques au boîtier électronique intégré (210) comprend l'incorporation de la pluralité d'éléments de localisation magnétiques dans le boîtier électronique intégré (210),
dans lequel l'incorporation de la pluralité d'éléments de localisation magnétiques dans le boîtier électronique intégré (210) comprend l'incorporation d'une pluralité de capteurs de localisation magnétiques à semi-conducteurs (300) dans le boîtier électronique intégré (210).

7. Procédé selon la revendication 6, dans lequel le boîtier électronique intégré (210) comprend un circuit intégré spécifique à l'application.
